# EUROPÄISCHE PATENTSCHRIFT

(11) **EP 1 411 871 B1**
(45) Veröffentlichungstag und Bekanntmachung des Hinweises auf die Patenterteilung: **29.12.2004**
(21) Anmeldenummer: 02762424.6
(22) Anmeldetag: 02.08.2002
(51) Int. Cl.: A61F 2/44

(54) **PLATZHALTER MIT VERÄNDERBARER AXIALER LÄNGE**
SPACER HAVING A VARIABLE AXIAL LENGTH
ELEMENT D'ECARTEMENT DE LONGUEUR AXIALE MODIFIABLE

(30) Priorität: 03.08.2001 DE 10138079
(43) Veröffentlichungstag der Anmeldung: 28.04.2004
(73) Patentinhaber: BIEDERMANN MOTECH GmbH, 78054 VS-Schwenningen (DE)
(72) Erfinder: BIEDERMANN, Lutz, 78048 VS-Villingen (DE); HARMS, Jürgen, 76227 Karlsruhe (DE); OSTERMANN, Peter, 46397 Bocholt (DE)
(74) Vertreter: Hofer, Dorothea, Dipl.-Phys.
(86) Internationale Anmeldenummer: PCT/EP2002/008648
(87) Internationale Veröffentlichungsnummer: WO 2003/013399

(56) Entgegenhaltungen:
- EP-A- 1 080 703
- WO-A-99/63913
- US-B1- 6 200 348

## Beschreibung

Die Erfindung betrifft einen Platzhalter zum Einsetzen zwischen zwei Wirbelkörper mit einer veränderbaren axialen Länge, der ein hülsenartiges erstes Teil und ein in diesem geführtes und in axialer Richtung relativ zum ersten Teil bewegbares zweites Teil zum Einstellen einer Gesamtlänge aufweist.

Ein derartiger Platzhalter ist aus EP 0 977 528 A 1 und US 6 200 348 B1 bekannt. Bei diesem werden die beiden Teile in der zusammengeschobenen Stellung zwischen zwei Wirbelkörper eingefügt und dann auf die gewünschte Länge von Hand auseinandergezogen und in der auseinandergezogenen Stellung dann arretiert.

Aufgabe der Erfindung ist es, einen Platzhalter der eingangs beschriebenen Art zu schaffen, bei dem der Operateur das Einstellen auf die gewünschte Länge auf einfachste Weise bewerkstelligen kann.

Diese Aufgabe wird durch einen Platzhalter der eingangs beschriebenen Art gelöst, der dadurch gekennzeichnet ist, daß die beiden Teile durch einen Hebel miteinander verbunden sind, dessen einer Drehpunkt mit dem einen Teil und dessen anderer Drehpunkt mit dem anderen Teil verbunden ist.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen:
- Fig. 1: eine Seitenansicht des Platzhalters in zusammengezogener Stellung;
- Fig. 2: einen Schnitt entlang der Linie II/II in Fig. 1;
- Fig. 3: den gleichen Schnitt wie Fig. 2, bei dem jedoch der Platzhalter auf seine Maximallänge auseinander gezogen ist;
- Fig. 4: eine Draufsicht auf den in Fig. 1 gezeigten Gegenstand;
- Fig. 5: eine zweite Ausführungsform in einer der Schnittdarstellung II/II entsprechenden Darstellung in auseinandergezogener Stellung;
- Fig. 6: die zweite Ausführungsform in zusammengezogener Darstellung;
- Fig. 7: einen Schnitt durch eine weitere Ausführungsform.

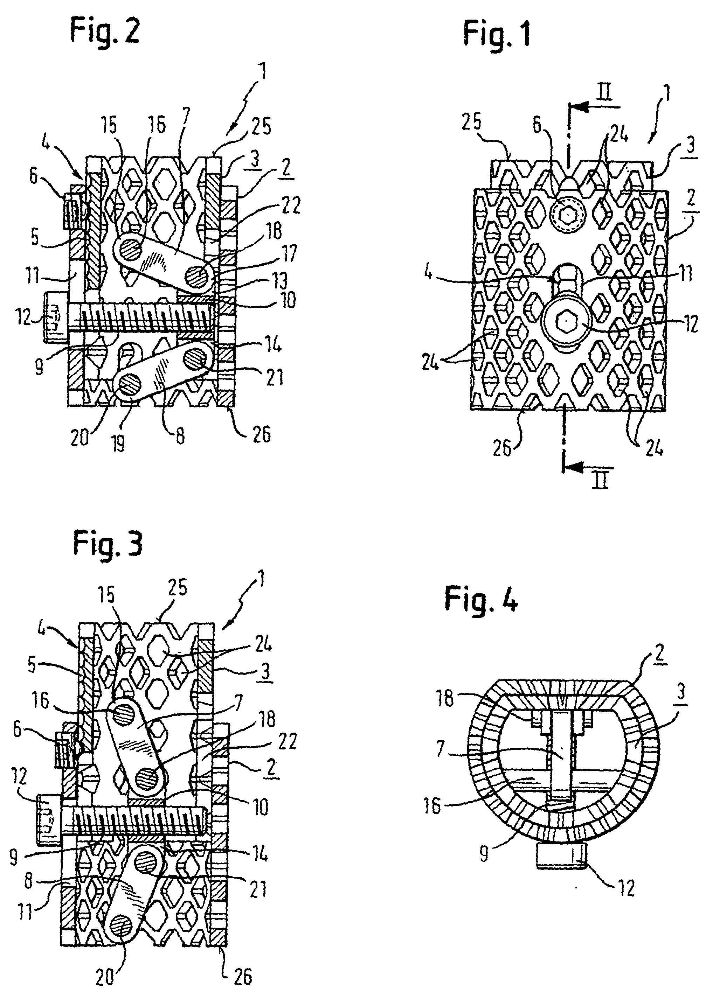

Wie am besten aus Fig. 1 ersichtlich ist, umfaßt ein Platzhalter 1 ein hülsenartiges erstes Teil 2 und ein in diesem geführtes hülsenartiges zweites Teil 3. Die beiden Teile können maximal ineinander geschoben werden, wie es in Fig. 1 und 2 gezeigt ist, und auf eine maximale Länge auseinanderbewegt werden, wie dies in Fig. 3 gezeigt ist.

Wie aus den Figuren 1 bis 3 ersichtlich ist, weist das innere zweite Teil 3 auf seiner dem äußeren ersten Teil 2 zugewandten Außenwand einen sich in axialer Richtung erstreckenden Abschnitt mit einer Rasterung 4 mit einer Mehrzahl von in axialer Richtung benachbart zueinander angeordneten und aneinander grenzenden Vertiefungen 5 und das äußere erste Teil 2 ein mit der Rasterung in Eingriff bringbares Feststellteil 6 auf. Das Feststellteil dient dazu, die beiden Teile in einer gewünschten Position zueinander zu arretieren.

Wie am besten aus den Figuren 2 und 4 ersichtlich ist, weist der Platzhalter 1 eine Hebeleinrichtung zum Verstellen der axialen Position der beiden Teile zueinander auf. In der ersten Ausführungsform umfaßt diese einen ersten Hebelarm 7, einen zweiten Hebelarm 8, eine Stellschraube 9 und eine Gewindehülse 10. Wie am besten aus Fig. 2 ersichtlich ist, weist das äußere erste Teil 2 seitlich eine Ausnehmung 11 auf, deren seitliche Ausdehnung kleiner ist als der Durchmesser des Kopfes 12 der Stellschraube 9, wie dies am besten aus Fig. 1 ersichtlich ist. Die Stellschraube 9 ist in der in Fig. 2 ersichtlichen Weise in den Innenraum der hülsenartigen Teile quer zur Axialrichtung eingefügt. Die Gewindehülse 10 ist auf der Schraube aufgeschraubt. Sie weist an ihren beiden sich in axialer Richtung der Hülse erstreckenden beiden Seiten Ansätze 13, 14 auf. Der Ansatz 13 dient zur Verbindung mit dem ersten Hebelarm 7. Der Hebelarm ist mit seinem ersten Ende 15 mittels einer an zwei gegenüberliegenden Stellen der Wandung des zweiten Teiles 3 lagernden Welle 16 um diese schwenkbar gelagert. Die Welle erstreckt sich senkrecht zur Längsachse des Platzhalters. Der erste Hebelarm ist an seinem der Verbindung mit der Welle 16 gegenüberliegenden zweiten Ende 17 über einen Bolzen bzw. eine Welle 18 mit dem Ansatz 13 um die Welle 18 schwenkbar verbunden. Der Bolzen bzw. die Welle 18 erstreckt sich parallel zur Welle 16.

Wie in Fig. 2 dargestellt ist, ist der zweite Hebelarm 8, um die Stellschraube 9 gesehen, symmetrisch zum ersten Hebelarm 7 ausgebildet bzw. angeordnet. An seinem ersten Ende 15 entsprechend gegenüber liegenden Ende 19 ist der Hebelarm über eine Welle 20 um diese schwenkbar gelagert. Die Welle 20 ist in den einander gegenüber liegenden Seitenwandungen des ersten Teiles 2 seitlich gelagert und erstreckt sich parallel zur Welle 16. An seinem dem Ende 19 gegenüber liegenden Ende ist der zweite Hebelarm mittels eines Bolzens bzw. einer Welle 21 mit dem Ansatz 14 um die Welle 21 schwenkbar verbunden.

Wie aus den Figuren 1 und 2 ersichtlich ist, ist die Ausnehmung 11 als ein sich in seiner Richtung parallel zur Längsachse des Platzhalters erstreckendes Langloch ausgebildet. Das Langloch ist so positioniert, daß die Stellschraube 9 in diesem so weit hin und her bewegbar ist, daß die Schraube von der in Fig. 2 gezeigten zusammengefahrenen Position bis zu der in Fig. 3 gezeigten auseinanderbewegten Position im Langloch hin und her bewegbar ist.

Im Betrieb wird der Platzhalter in der in Fig. 2 gezeigten zusammen gezogenen Stellung mit minimaler Länge in axialer Richtung zwischen die Wirbel eingesetzt. Dann wird die Länge durch Eingreifen mittels eines Schraubendrehers in eine entsprechende Schlitz- bzw. Sechskantöffnung des Kopfes 12 der Stellschraube 9 dadurch auf eine gewünschte Länge verstellt, daß die Stellschraube 9 so gedreht wird, daß die Gewindehülse 10 aus der in Fig. 2 gezeigten äußersten Position, in der sich die Gewindehülse am freien Ende der Stellschraube 9 befindet, zum Kopf hin bewegt wird. Dabei werden die beiden Hebel 7,8 aus ihrer zusammengefahrenen Position in eine in Fig. 3 gezeigte maximal gestreckte Position verfahren. Auf diese Weise werden die beiden hülsenartigen Teile 2 und 3 aus der in Fig. 2 gezeigten zusammen gezogenen Stellung in die in Fig. 3 gezeigte expandierte Stellung oder jede Zwischenstellung bewegt. Durch die Schraubenführung zwischen Stellschraube 9 und Gewindehülse 10 bleiben die beiden Teile zunächst in der durch das Drehen der Stellschraube 9 erreichten Stellung. Sobald diese Stellung als endgültig angesehen wird, erfolgt ein vollständiges Arretieren durch Anziehen der das Feststellteil 6 bildenden Arretierschraube, die zu diesem Zweck in eine Vertiefung 5 der Rasterung 4 eingreift.

Bei der oben beschriebenen Ausführungsform ist die Länge der Stellschraube 9 so gewählt, daß die Stellschraube mit ihrem freien Ende in den hohlen Innenraum des zweiten Teiles 3 hineinreicht, ohne mit der gegenüber liegenden Wandung des zweiten Teiles 3 in Eingriff zu gelangen, so daß keine Behinderung der Bewegung des zweiten Teiles 3 erfolgt. Wie in den Figuren gezeigt ist, weist das zweite Teil 3 bevorzugt eine Ausnehmung 22 auf, die als Langloch ausgebildet ist, welches sich in seiner Längsrichtung parallel zur Längsachse des Platzhalters erstreckt und die in ihrer Länge und Breite so ausgebildet ist, daß das freie Ende der Stellschraube 9 mit der darauf gleitenden Gewindehülse 10 und den beiden Ansätzen 13 und 14 und den damit verbundenen Enden der beiden Hebelarme 7 und 8 bei der in den Figuren 2 und 3 gezeigten Hinundher-Bewegung in dem Langloch frei hin und her bewegen können. Auf diese Weise wird erreicht, daß die Stellschraube 9 eine größere Länge aufweisen kann, wodurch der Weg der Gewindehülse 10 vergrößert und damit die Expansionsmöglichkeit der beiden Teile bzw. des Platzhalters vergrößert wird.

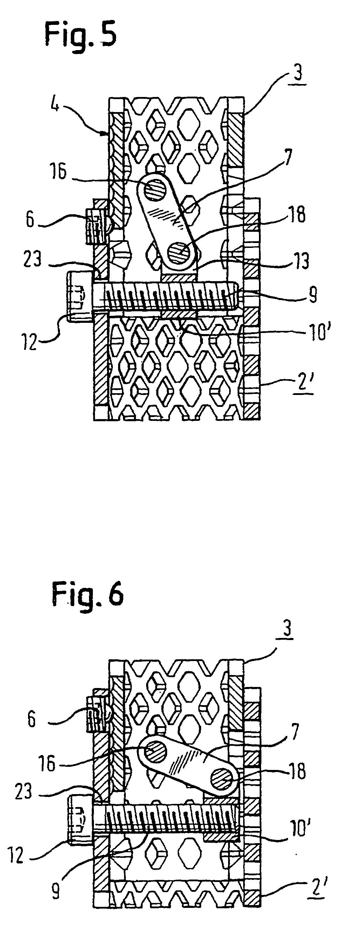

In einer in den Figuren 5 und 6 gezeigten abgewandelten Ausführungsform sind das zweite Teil 3, die Stellschraube 9, der erste Hebelarm 7, der Ansatz 13 und die beiden Wellen 16 und 18 in gleicher Weise ausgebildet wie die entsprechenden Elemente in der ersten Ausführungsform.

In der zweiten Ausführungsform ist anstelle des Langloches 11 ein die Stellschraube führendes Rundloch 23 in der Wandung des ersten Teiles 2' vorgesehen, welches in seinem Durchmesser so gewählt ist, daß die Stellschraube in diesem Loch drehbar geführt ist. Die Gewindehülse 10' weist nur einen Ansatz 13 auf.

Im Betrieb erfolgt das Einstellen zwischen der in Fig. 6 gezeigten zusammengeschobenen Stellung und der in Fig. 5 gezeigten expandierten Stellung wie bei der ersten Ausführungsform durch Drehen der Stellschraube 9 derart, daß die Gewindehülse 10' aus der in Fig. 6 gezeigten äußersten Stellung am freien Ende zum Kopf hin soweit verschraubt wird, bis die Expansion auf ein gewünschtes Maß erfolgt ist bzw. der Hebel 7 nahezu in die vertikale Position bewegt ist. Das abschließende Arretieren erfolgt wie beim ersten Ausführungsbeispiel durch Festziehen einer Feststellschraube 6 in Kooperation mit den Vertiefungen 5 der Rasterung 4.

Wie aus den Figuren ersichtlich ist, sind die beiden Wandungen des ersten und zweiten Teiles jeweils so ausgebildet, daß sie in Umfangsrichtung verteilt eine Vielzahl von rautenförmigen Ausnehmungen 24 aufweisen. Die einander gegenüber liegenden freien Enden 25, 26 sind, wie in den Figuren gezeigt ist, jeweils zackenförmig ausgebildet, wodurch ein drehstabilisierendes Eingreifen in die daran angrenzenden Wirbelkörperwandungen erleichtert wird. Die Ausnehmungen in der Wandung erleichtern das Einwachsen nach der Operation.

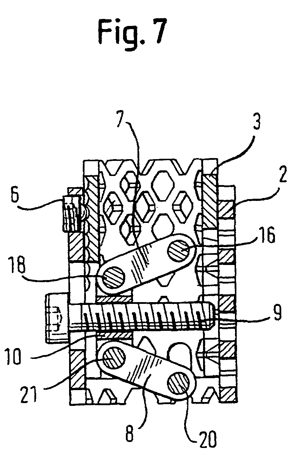

In den oben beschriebenen Ausführungsbeispielen ist die Verstelleinrichtung der Stellschraube 9, Gewindehülse 10 und Hebelarmen 7, 8 bzw. 7 jeweils so ausgebildet, daß die maximale Extension erfolgt, wenn die Gewindehülse 10 maximal zu dem Kopft 12 hinbewegt ist, und weitmöglichst zusammengeschoben, wenn die Gewindehülse 10 ihren größtmöglichen Abstand vom Kopf 12 besitzt. Es ist aber auch möglich, diese Einrichtung dahingehend umzukehren, daß die größtmögliche Extension erreicht wird, wenn die Gewindehülse 10 ihren größten Abstand vom Kopf 12 aufweist. Bei kleinstem Abstand vom Kopf 12 weist die Höhe ihren kleinstmöglichen Wert auf. Ein solches Ausführungsbeispiel ist in Figur 7 beschrieben.

## Patentansprüche

1. Platzhalter (1) zum Einsetzen zwischen zwei Wirbelkörper mit einer veränderbaren axialen Länge, mit einem hülsenartigen ersten Teil (2) und einem in diesem geführten und in axialer Richtung relativ zum ersten Teil (2) bewegbaren zweiten Teil (3) zum Einstellen einer Gesamtlänge, **dadurch gekennzeichnet, daß** die beiden Teile (2,3) durch einen Hebel (7,8) miteinander verbunden sind, dessen einer Drehpunkt (16) mit dem einen Teil (3) und dessen anderer Drehpunkt (18) wirkungsmäßig mit dem anderen Teil (2) verbunden ist.

2. Platzhalter nach Anspruch 1, **dadurch gekennzeichnet, daß** der Hebel (7,8) zweiarmig ausgebildet ist, wobei die beiden den einen Drehpunkten (16,20) abgewandten Enden mit einer auf einer in einem Langloch (11) der Wandung des ersten Teils (2) geführten Stellschraube (9) geführten Gewindehülse (10) verbunden sind.

3. Platzhalter nach Anspruch 1, **dadurch gekennzeichnet, daß** der andere Drehpunkt (18) mit einer auf einer in einer Bohrung (23) der Wandung des ersten Teils (2) geführten Stellschraube geführten Gewindehülse (10') verbunden ist.

4. Platzhalter nach einem der Ansprüche 2 bis 3, **dadurch gekennzeichnet, daß** auf der dem Langloch (11) oder der Bohrung (23) gegenüber liegenden Seite die Wandung des zweiten Teiles (3) eine sich in ihrer Längsrichtung parallel zur Längsachse des Platzhalters erstreckende Längsausnehmung aufweist, in die das freie Ende der Stellschraube (9) frei hineinragt.

5. Platzhalter nach einem der Ansprüche 1 bis 4, **dadurch gekennzeichnet, daß** das zweite Teil (3) auf seiner dem ersten Teil (2) zugewandten Außenwand einen sich in axialer Richtung erstreckenden Abschnitt mit einer Rasterung (4) mit einer Mehrzahl von in axialer Richtung benachbart zueinander angeordneten und aneinandergrenzenden Vertiefungen (5) und das erste Teil (2) ein mit der Rasterung (4) zum Arretieren in einer gewünschten Länge zusammenwirkendes Feststellteil (6) aufweist.

6. Platzhalter nach einem der Ansprüche 1 bis 4, **dadurch gekennzeichnet, daß** die Wandungen des ersten und zweiten Teiles (2,3) über die Oberfläche verteilte Ausnehmungen (24) zur Verbesserung des Einwachsen aufweisen.

## Claims

1. Spacer (1) to be inserted between two vertebral bodies which has a variable axial length, which has a cylinder-shaped first element (2) and a second element (3) guided therein that can be axially displaced relative to the first element (2) for adjusting an overall length, **characterised in that** both parts (2, 3) are interlinked by a lever (7, 8), one fulcrum (16) of the lever being functionally linked with the one element (3) and the other fulcrum (18) being functionally linked with the other element (2).

2. Spacer according to claim 1, **characterised in that** the lever (7, 8) is designed with two arms, whereby the two ends facing away from the one fulcrum (16, 20) are linked with a threaded bush (10) guided on an adjustment screw (9) guided in a long hole (11) of the wall of the first element (2).

3. Spacer according to claim 1, **characterised in that** the other fulcrum (18) is connected with a threaded bush (10') guided on an adjustment screw guided in a bore hole (23) of the wall of the first element (2).

4. Spacer according to one of claims 2 to 3, **characterised in that** on the side opposite the long hole (11) or the bore hole (23), the wall of the second element (3) has a longitudinal recess extending in its longitudinal direction parallel to the longitudinal axis of the spacer, in which the free end of the adjustment screw (9) freely projects.

5. Spacer according to one of claims 1 to 4, **characterised in that** the second element (3) on its outer wall facing towards the first element (2) has a section extending axially with a screen (4) with a multiplicity of recesses (5) disposed axially adjacent to each other and contiguous with each other and the first element (2) has a locking element (6) co-operating with the screen (4) to act as a stop at any desired length.

6. Spacer according to one of claims 1 to 4, **characterised in that** the walls of the first and second element (2, 3) have recesses (24) distributed over the surface to improve integration.

## Revendications

1. Élément d'écartement (1) de longueur axiale modifiable à insérer entre deux corps vertébraux, avec une première partie en forme de douille (2) et une seconde partie (3) guidée dans celle-ci et mobile dans le sens axial par rapport à la première partie (2) pour l'ajustement d'une longueur totale, **caractérisé en ce que** les deux parties (2, 3) sont reliées entre elles par un levier (7, 8) dont un point de rotation (16) est relié à la partie (3) et l'autre point de rotation (18) est en liaison active avec l'autre partie (2).

2. Élément d'écartement selon la revendication 1, **caractérisé en ce que** le levier (7, 8) est formé de deux bras, les deux extrémités éloignées des premiers points de rotation (16, 20) étant reliées avec une douille filetée (10) guidée sur une vis de réglage (9) passant dans un trou allongé (11) dans la paroi de la première partie (2).

3. Élément d'écartement selon la revendication 1, **caractérisé en ce que** l'autre point de rotation (18) est relié à une douille filetée (10') guidée sur une vis de réglage passant dans un trou (23) dans la paroi de la première partie (2).

4. Élément d'écartement selon l'une des revendications 2 à 3, **caractérisé en ce que** sur le côté faisant face au trou allongé (11) ou au trou (23), la paroi de la seconde partie (3) présente un évidement allongé s'étendant dans son sens longitudinal parallèlement à l'axe longitudinal de l'élément d'écartement, dans lequel s'étend librement l'extrémité libre de la vis de réglage (9).

5. Élément d'écartement selon l'une des revendications 1 à 4, **caractérisé en ce que** la seconde partie (3) possède sur sa paroi extérieure orientée vers la première partie (2) une section s'étendant dans le sens axial avec une trame (4) pourvue d'une pluralité de renfoncements (5) voisins les uns des autres dans le sens axial et contigus les uns aux autres et la première partie (2) possède un élément d'arrêt (6) coopérant avec la trame (4) pour le blocage à une longueur souhaitée.

6. Élément d'écartement selon l'une des revendications 1 à 4, **caractérisé en ce que** les parois de la première partie et de la seconde (2, 3) présentent des évidements (24), répartis sur leur surface, destinés à améliorer l'ostéo-intégration.
